# EUROPEAN PATENT SPECIFICATION

(11) **EP 2 864 499 B1**
(45) Date of publication and mention of the grant of the patent: **07.11.2018**
(21) Application number: 13742259.8
(22) Date of filing: 21.06.2013
(51) Int. Cl.: C12Q 1/6883

(54) **BIOMARKERS FOR DETERMINING THE M. TUBERCULOSIS INFECTION STATUS**
BIOMARKER ZUR BESTIMMUNG DES M. TUBERCULOSIS-INFEKTIONSSTATUS
BIOMARQUEURS PERMETTANT DE DÉTERMINER L'ÉTAT D'INFECTION PAR M. TUBERCULOSIS

(30) Priority: 22.06.2012 GB 201211158
(43) Date of publication of application: 29.04.2015
(73) Proprietor: CompanDX Limited, Nottingham NG1 1GF (GB)
(72) Inventor: BALL, Graham Roy, Nottingham NG11 8NS (GB)
(74) Representative: Potter Clarkson LLP
(86) International application number: PCT/GB2013/051635
(87) International publication number: WO 2013/190321

(56) References cited:
- WO-A1-2004/001070
- WO-A2-2006/125973
- WO-A2-2009/158521
- WO-A2-2011/066008
- JOEL N. H. STERN ET AL: "Molecular signatures distinguishing active from latent tuberculosis in peripheral blood mononuclear cells, after in vitro antigenic stimulation with purified protein derivative of tuberculin (PPD) or Candida: a preliminary report", IMMUNOLOGIC RESEARCH, vol. 45, no. 1, 22 July 2008 (2008-07-22), pages 1-12, XP055029887, ISSN: 0257-277X, DOI: 10.1007/s12026-008-8024-2

### SEQUENCE LISTING

<110> Nottingham Trent University
<120> Biomarkers AND USES THEREOF
<130> JA63828P.WOP
<150> GB1211158.9
   <151> 2012-06-22
<160> 35
<170> PatentIn version 3.5
<210> 1
   <211> 50
   <212> DNA
   <213> Homo sapiens
<400> 1
   gagaccctga ctctacgaaa attaaaagtt agcccggtgt ggtggcgcgc 50
<210> 2
   <211> 50
   <212> DNA
   <213> Homo sapiens
<400> 2
   cgcttgcatg ctcacctctg gcagcagggc agtcacggct ccgccatgga 50
<210> 3
   <211> 50
   <212> DNA
   <213> Homo sapiens
<400> 3
   ggcaggatga ttgcttaagc ccaggagttc ggggttacag tgagctgtgg 50
<210> 4
   <211> 50
   <212> DNA
   <213> Homo sapiens
<400> 4
   gagggcaagc caccaaatta cctaggctga ggttagagag attggccagc 50
<210> 5
   <211> 50
   <212> DNA
   <213> Homo sapiens
<400> 5
   gagctcgcct atgagcaagt ggcaaaggcc ctcaaataag cccctcctgg 50
<210> 6
   <211> 50
   <212> DNA
   <213> Homo sapiens
<400> 6
   atactggagg ggcagtgggt gccagtagtg tcctgctggt ccttcctgaa 50
<210> 7
   <211> 50
   <212> DNA
   <213> Homo sapiens
<400> 7
   ccatggtgat ggatggtttg gaaagggaat gttggtgcct tttgtgccac 50
<210> 8
   <211> 50
   <212> DNA
   <213> Homo sapiens
<400> 8
   tgtggtgaag aggcagaacc gaggtcggga gcccattgat gtctgagcct 50
<210> 9
   <211> 50
   <212> DNA
   <213> Homo sapiens
<400> 9
   ccccagtgtg tataagctgg catttcgcca gcttgtacgt agcttgccac 50
<210> 10
   <211> 50
   <212> DNA
   <213> Homo sapiens
<400> 10
   gggaagagag tttgcaggcc tgtgcttcaa agaactcttc tagtctgctt 50
<210> 11
   <211> 50
   <212> DNA
   <213> Homo sapiens
<400> 11
   gcccttcacc attgtggaat gatgccctgg ctttaaggtt tagctccaca 50
<210> 12
   <211> 50
   <212> DNA
   <213> Homo sapiens
<400> 12
   tgagatctac aagttgaccc gcaaggaggc ccaggagtag gaggctgcag 50
<210> 13
   <211> 50
   <212> DNA
   <213> Homo sapiens
<400> 13
   gaagggtccc cctgctgact ggagagctgg gaatatggca tttggacact 50
<210> 14
   <211> 50
   <212> DNA
   <213> Homo sapiens
<400> 14
   gacagatttg ttcatagacc ctctcaagtg ccctctccga gctggtaggc 50
<210> 15
   <211> 50
   <212> DNA
   <213> Homo sapiens
<400> 15
   gcctcttctc tcaagcctgc ttcagatcat aagttcttcc acacatctcc 50
<210> 16
   <211> 50
   <212> DNA
   <213> Homo sapiens
<400> 16
   gtgccgagct gtccatagct gcagtgaaag gtgaagagca agaccttctc 50
<210> 17
   <211> 50
   <212> DNA
   <213> Homo sapiens
<400> 17
   gcttctccag atccccagcg ccaggagtgg tttgcccggt acttcacatt 50
<210> 18
   <211> 50
   <212> DNA
   <213> Homo sapiens
<400> 18
   cctttatttt gctgcttgat gatgagaggg agggctgctg ccacagactg 50
<210> 19
   <211> 50
   <212> DNA
   <213> Homo sapiens
<400> 19
   atggagctgt ctttcagatc tttcctggga ttacccctgc ctacccccag 50
<210> 20
   <211> 50
   <212> DNA
   <213> Homo sapiens
<400> 20
   tgcagccgtc catagcagta cccctaaaat cccaccagaa tacgggtccc 50
<210> 21
   <211> 50
   <212> DNA
   <213> Homo sapiens
<400> 21
   caccagaagt agcagagaag cagggggcca gagctacaac agtattcttc 50
<210> 22
   <211> 50
   <212> DNA
   <213> Homo sapiens
<400> 22
   acttctactt gccaacatct gccttgctgg acttgtatgg gattgtctcc 50
<210> 23
   <211> 50
   <212> DNA
   <213> Homo sapiens
<400> 23
   agtgaccatt gaaaatcacc ctcatctgcc accacctccc atgtgttcag 50
<210> 24
   <211> 50
   <212> DNA
   <213> Homo sapiens
<400> 24
   ttgtcatcct ttgagctgct gactgccaat aacattcttc gatctctggg 50
<210> 25
   <211> 50
   <212> DNA
   <213> Homo sapiens
<400> 25
   gtcgctcgga cccaggacac tacagtttct ctatgcgatc tccagagctc 50
<210> 26
   <211> 50
   <212> DNA
   <213> Homo sapiens
<400> 26
   accgggacat ccggctgatg gtcatggaga tccgcaatgc ttatgctgtg 50
<210> 27
   <211> 50
   <212> DNA
   <213> Homo sapiens
<400> 27
   ggacattaag ttcagtggcc atgttagctg ggtcgggaag acatccatgg 50
<210> 28
   <211> 50
   <212> DNA
   <213> Homo sapiens
<400> 28
   tggaagatca gacccagctc cttacccttg tctgccagtt gtaccagggc 50
<210> 29
   <211> 50
   <212> DNA
   <213> Homo sapiens
<400> 29
   atactggagg ggcagtgggt gccagtagtg tcctgctggt ccttcctgaa 50
<210> 30
   <211> 50
   <212> DNA
   <213> Homo sapiens
<400> 30
   ccatggtgat ggatggtttg gaaagggaat gttggtgcct tttgtgccac 50
<210> 31
   <211> 50
   <212> DNA
   <213> Homo sapiens
<400> 31
   ggtccccatg tgcctgttgt tcagccctct ctcttgttcc ctttctgagc 50
<210> 32
   <211> 50
   <212> DNA
   <213> Homo sapiens
<400> 32
   gaaccaggag acacgtgtgg cgggcagtgg gacccataga tcttctgaaa 50
<210> 33
   <211> 50
   <212> DNA
   <213> Homo sapiens
<400> 33
   gtaacggagt ttagagccag ggctgatgct ttggtgtggc cagcactctg 50
<210> 34
   <211> 50
   <212> DNA
   <213> Homo sapiens
<400> 34
   aaggctcgat cctgcgagaa ggaagtggtc tctgcccagc ctgccacctt 50
<210> 35
   <211> 50
   <212> DNA
   <213> Homo sapiens
<400> 35
   cctcaacagg cccagggagg gaagtgtgag cgccttggta tgacttaaaa 50

## Description

The present invention relates to novel biomarkers for determining the *Mycobacterium tuberculosis* infection status of a subject, and to uses of novel panels of biomarkers.

Tuberculosis (TB) is an infection caused by *Mycobacterium tuberculosis (M. tuberculosis)* and is a major cause of morbidity and mortality worldwide. *M. tuberculosis* is an airborne bacterial infection that primarily affects the lungs. It is estimated that approximately 2.2 billion people or a third of the world's population are infected with *M. tuberculosis.*

The majority of infected people remain asymptomatic. Infected people who remain asymptomatic are said to have latent *M. tuberculosis* infection (latent TB). A person infected with M. tuberculosis has about a 10% lifetime risk of developing active *M. tuberculosis* infection (active TB) where symptoms of *M. tuberculosis* infection are shown.

*M. tuberculosis* is a substantial management and cost burden for healthcare systems and could be reduced with improvements in diagnosis and informed patient management. Treatments for *M. tuberculosis* infection vary depending on the type of *M. tuberculosis* infection status that a person has. A person who has latent *M. tuberculosis* infection and does not have active *M. tuberculosis* infection may be given preventative therapy.

Current tests for *M. tuberculosis* infection cannot distinguish between latent and active *M. tuberculosis* infection and cannot identify which individuals having latent *M. tuberculosis* infection will go on to develop active *M. tuberculosis* infection.

It would be advantageous to be able to test for the infection status of an individual subject to distinguish between uninfected individuals and individuals having latent *M. tuberculosis* infection and active *M. tuberculosis* infection. Such a test could also be used to follow the infection status of individuals to determine whether TB is being activated or during treatment to determine when the *M. tuberculosis* infection has been cleared.

WO2004001070 A1, WO2009158521 A1, WO 2011066008 A2, WO 2006125973 and Joel Stern et al., Immunol. Res. 45, pp. 1-12, 2009, describes markers for evaluating a tuberculosis infection.

The invention is as set out in the claims.

A first aspect of the invention provides a method of determining the *M. tuberculosis* infection status of a subject comprising determining the expression level of NXNL1 and one or more genes selected from the group consisting of: PSMA7, C6orf61 and EMP1 in a sample from a subject,
wherein a decreased expression level of the gene NXNL1; and a decreased expression level of PSMA7 or C6orf61 or an increased expression level of the gene EMP1, differentiates between subjects not infected with M. tuberculosis and subjects with latent M. tuberculosis and indicates that a subject has a latent M. tuberculosis infection,
wherein the sample is a sample of blood, optionally whole blood, blood plasma or blood serum; sputum; saliva; wound exudate; urine; faeces; peritoneal fluid; or a respiratory section.

Other disclosures provide a method of determining the *M. tuberculosis* infection status of a subject comprising:
(a) providing a sample of material obtained from the subject; and
(b) determining the expression level of one or more genes selected from the group consisting of: NXNL1, PSMA7, C6orf61, EMP1, CLIC1, LACTB and DUSP3 in the sample.
Optionally, the method could comprise a step (c) determining the expression level of one or more genes selected from table 3 or table 4.

The method may further comprise: (d) employing the expression level determined in (b) and optionally (c) to distinguish between subjects not infected with *M*. *tuberculosis*, subjects with latent *M. tuberculosis* infection and subjects with active *M. tuberculosis* infection.

Subjects not infected with *M. tuberculosis* may be defined as subjects that test negative for *M. tuberculosis* using a tuberculin-skin test (TST) and/or test negative for *M. tuberculosis* using an antigen-specific IFN-gamma release assay (IGRA).

Subjects with latent *M. tuberculosis* infection may be defined as subjects that test positive for *M. tuberculosis* using a tuberculin-skin test (TST) and/or test positive for *M. tuberculosis* using an antigen-specific IFN-gamma release assay (IGRA) but do not have symptoms of tuberculosis such as a persistent cough.

Subjects with active *M. tuberculosis* infection may be confirmed by culture for *M. tuberculosis* from a blood, serum or sputum sample and also test positive for *M. tuberculosis* using a tuberculin-skin test (TST) and/or test positive for *M. tuberculosis* using an antigen-specific IFN-gamma release assay (IGRA) and also have symptoms of tuberculosis.

The sample of material is a sample of blood, sputum, saliva, wound exudate, urine, faeces, peritoneal fluid or any respiratory secretion. A sample of blood may be whole blood, blood plasma or blood serum.

Preferably the sample is a sample of whole blood. Blood samples have the advantage that they are readily obtainable and tend to be more homogenous in nature than other sample types. Samples of whole blood contain RNA which can be extracted to generate a transcriptional profile.

The expression level of one or more genes selected from the group consisting of: NXNL1, PSMA7, C6orf61, EMP1, CLIC1, LACTB and DUSP3 or selected from table 3 or table 4 in a sample may be determined by any suitable method. Such methods include methods that quantify the nucleotide products of these genes such as: quantitative PCR using suitable oligonucleotide primers designed to adhere within the sequence of an mRNA encoded by the gene of interest; analysis of expression arrays; next generation sequencing, comparative genomic hybridisation arrays (CGH arrays); multiplexed PCR. The expression level of one or more genes selected from the group consisting of: NXNL1, PSMA7, C6orf61, EMP1, CLIC1, LACTB and DUSP3 or selected from table 3 or table 4 in a sample may also be determined using methods that quantify the protein products of the selected genes, for example: ELISA immunohistochemistry; protein aptamer arrays or protein immunological arrays.

The expression level of the gene may be determined by measuring the rate of polymerisation of the RNA using standard techniques.

In one disclosure the method may not include the step of obtaining the sample.

In one disclosure the method may include a further step of comparing the determined value of the expression level of one or more genes selected from the group consisting of: NXNL1, PSMA7, C6orf61, EMP1, CLIC1, LACTB and DUSP3 or selected from table 3 or table 4 in a sample with a reference value.

The reference value may be the value for the expression level of the same gene in a sample of the same sample type, from an individual who is known to have or not to have infection with *M. tuberculosis.* Alternatively, or additionally, the reference value may be the expression level of the same gene in the same sample type in a sample taken previously from the same subject, for example, prior to or during the course of a particular treatment. The reference sample may be a sample of the same type, for example, both samples may be blood samples. In this way the expression level of one or more genes selected from the group consisting of: NXNL1, PSMA7, C6orf61, EMP1, CLIC1, LACTB and DUSP3 or selected from table 3 or table 4 in a sample may be used to monitor the progression of an infection in a subject, and/or to monitor the efficacy of a particular treatment in a subject.

Alternatively or in addition the reference value may be the expression level for the selected gene in an individual that is infected with *M. tuberculosis* or the reference value may be the expression level for the selected gene in an individual that is not infected with *M. tuberculosis.*

The method may be carried out *in vitro.*

The subject may be a mammal, and is preferably a human, but may alternatively be a monkey, ape, cat, dog, cow, horse, deer, badger, rabbit or rodent.

The method may be used in one or more of the following; diagnosing whether or not a subject has *M. tuberculosis* infection; advising on the prognosis for a subject with a *M. tuberculosis* infection; and monitoring the effectiveness or response of a subject to a particular treatment for infection by *M. tuberculosis.*

In the method of the invention altered expression level of NXNL1 and one or more genes selected from: PSMA7, C6orf61 and EMP1 and optionally one or more genes selected from table 3 can differentiate between subjects not infected with *M*. *tuberculosis* and subjects with latent *M. tuberculosis* infection. NXNL1, PSMA7, C6orf61 and EMP1 and optionally one or more genes selected from table 3 may be used as a biomarker panel to distinguish between subjects not infected with *M*. *tuberculosis* and subjects with latent *M. tuberculosis* infection with a high degree of accuracy. This biomarker panel is advantageous because it provides a high degree of accuracy with only a small number of biomarkers. The evaluation error of this biomarker panel is 0.93%. Evaluation error is calculated as the square root of the averaged difference between model predictions and the actual TB status squared, expressed as a percentage.

In subjects with latent *M. tuberculosis* infection the expression levels of NXNL1, PSMA7 and C6orf61 are lower than in subjects not infected with *M. tuberculosis.* In subjects with latent *M. tuberculosis* infection the expression levels of EMP1 are higher than in subjects not infected with *M. tuberculosis.* Therefore, if these four markers are used together as a biomarker panel a pattern of decreased expression of NXNL1, PSMA7 and C6orf61 and also increased expression of EMP1 (compared to the level expected in a control subject not infected with *M. tuberculosis)* indicates that the subject may have latent *M. tuberculosis* infection.

In the method altered expression level of NXNL1 and one, two, or three of the genes PSMA7, C6orf61 and EMP1 may be evaluated together to differentiate between subjects not infected with *M. tuberculosis* and subjects with latent *M. tuberculosis* infection.

In order to improve the accuracy of the biomarker panel for distinguishing subjects that are not infected with *M. tuberculosis* and subjects with latent *M. tuberculosis* infection one, two or three biomarkers listed in table 3 may be tested in addition to NXNL1 and optionally PSMA7, C6orf61 and EMP1.

One, two or three of the following biomarkers may be tested in addition to NXNL1 and optionally PSMA7, C6orf61 and EMP1 wherein increased expression of LOC389541 and/or increased expression of MID1IP1 and/or increased expression of KLRC3 and/or increased expression of KLF9 and/or decreased expression GPR117 and/or increased expression of FBXO32 and/or decreased expression of TAZ and/or increased expression of C5ORF29 and/or decreased expression of HSDL1 and/or increased expression of CHUK and/or increased expression of LOC652062 and/or decreased expression of HIP1 and/or increased expression of C6ORF60 and/or increased expression of MTMR11 indicates that a subject has latent *M. tuberculosis* infection.

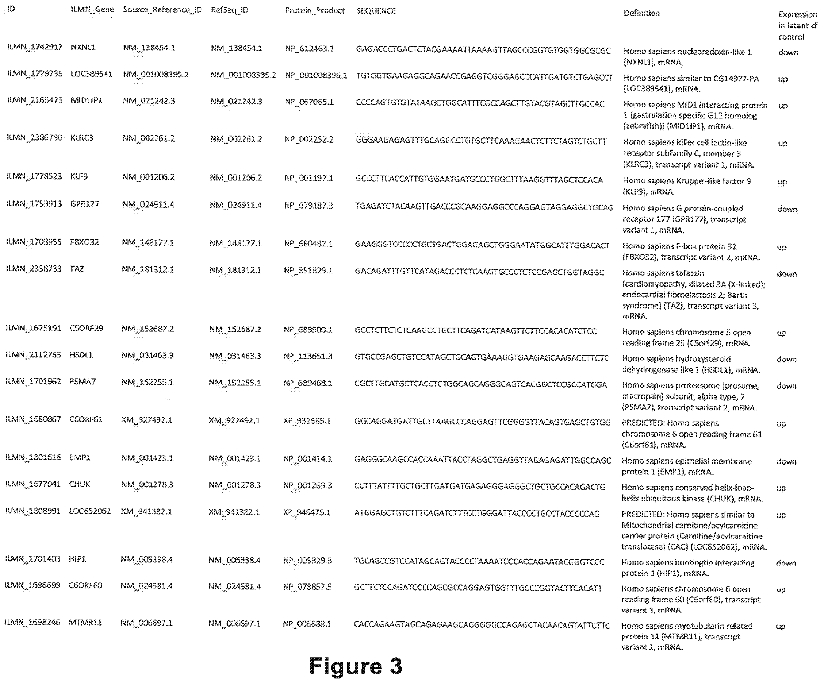

The full names and nucleotide sequences of each of the biomarkers useful in distinguishing between subjects that are not infected with *M. tuberculosis* and subjects with latent *M. tuberculosis* infection is shown in figure 3. The right hand column of figure 3 shows whether the biomarker expression is increased (up) or decreased (down) in subjects that have latent *M. tuberculosis* infection.

In one disclosure altered expression level of one or more genes selected from: CLIC1, LACTB and DUSP3 can differentiate between subjects with latent *M*. *tuberculosis* infection and subjects with active *M. tuberculosis* infection.

Altered expression level of one, two or all of the genes CLIC1, LACTB and DUSP3 may be evaluated together to differentiate between subjects with latent *M*. *tuberculosis* infection and subjects with active *M. tuberculosis* infection.

In one disclosure altered expression level of one or more genes selected from: CLIC1, LACTB and DUSP3 and optionally one or more genes selected from table 4 can differentiate between subjects with active *M. tuberculosis* infection and subjects with latent *M. tuberculosis* infection. CLIC1, LACTB and DUSP3 and optionally one or more genes selected from table 4 may be used as a biomarker panel to distinguish between subjects with active *M. tuberculosis* infection and subjects with latent *M. tuberculosis* infection with a high degree of accuracy. This biomarker panel is advantageous because it provides a high degree of accuracy with only a small number of biomarkers. The evaluation error of this biomarker panel is 0.26%.

In subjects with active *M. tuberculosis* infection the expression levels of CLIC1, LACTB and DUSP3 are higher than in subjects with latent *M. tuberculosis* infection or subjects not infected with *M. tuberculosis.* Therefore, if these three markers are used together as a biomarker panel a pattern of increased expression of CLIC1, LACTB and DUSP3 (compared to the level expected in a subject with latent *M. tuberculosis* infection or subjects not infected with *M. tuberculosis)* indicates that the subject may have active *M. tuberculosis* infection.

The sensitivity of the biomarker panel may be increased by additionally testing one or more biomarkers listed in table 4. If the level of any of the biomarkers in table 4 is increased this indicates that the subject may have active *M. tuberculosis* infection.

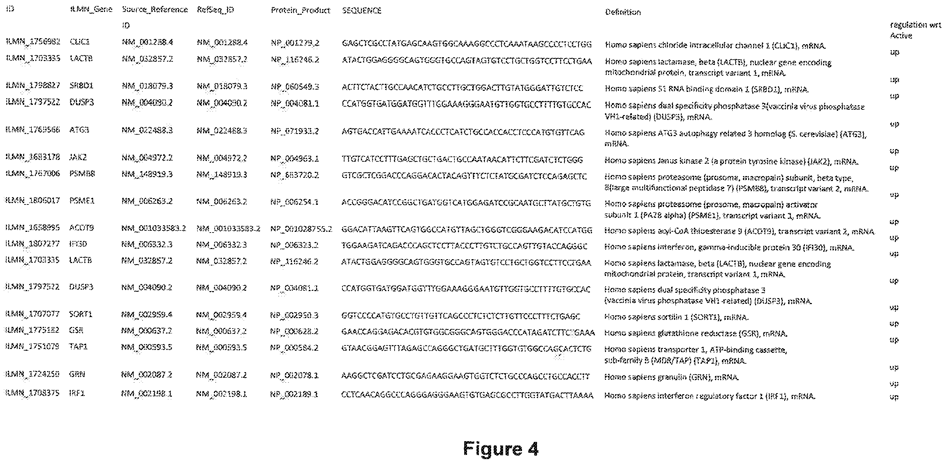

The full names and nucleotide sequences of each of the biomarkers useful for testing whether a subject has active *M. tuberculosis* infection or latent *M. tuberculosis* infection is shown in figure 4. The right hand column of figure 4 shows whether the biomarker expression is increased (up) or decreased (down) in subjects that have active *M. tuberculosis* infection.

A subject may be tested for expression levels of one, two, three, four, five, six, or all seven biomarkers selected from the group consisting of: NXNL1, PSMA7, C6orf61, EMP1, CLIC1, LACTB and DUSP3. This may allow the *M*. *tuberculosis* infection status of the subject to be determined as comparing the expression levels of a number of or all of these biomarkers may allow a subject to be classified as a subject not infected with *M. tuberculosis,* a subject with active *M. tuberculosis* infection or a subject with latent *M. tuberculosis* infection.

The altered expression level is an expression level that is higher or lower than the expression level expected in a subject not infected with *M. tuberculosis.* Higher or lower expression level means an expression level that is statistically significantly higher or lower than the control expression level that it is compared to. Statistical significance may be measured using standard statistical methods. Higher or lower expression level may be a statistically significantly higher or lower expression level when the significance is corrected using the number of samples.

The expression level of the selected gene may be determined using the level of mRNA encoded by that gene present in the sample.

The expression level of the selected gene may be determined using the level of mRNA encoded by that gene present in the sample.

The expression level of one or more further genes may also be determined.

A further disclosure provides a kit for use in determining the *M. tuberculosis* infection status in a subject comprising at least one agent for determining the expression level of one or more genes selected from the group consisting of: NXNL1, PSMA7, C6orf61, EMP1, CLIC1, LACTB and DUSP3 in a sample from a subject and instructions for determining the *M. tuberculosis* infection status of the subject. The instructions may include instructions to perform an assay for expression level of one or more of the selected genes. The instructions may provide reference values for comparison with values for the expression level of the selected gene.

The agent may an oligonucleotide, for example an oligonucleotide that adheres to a mRNA encoded by the selected gene. The kit may provide a pair of oligonucleotides suitable for amplifying the selected gene or an mRNA thereof. The expression level of one or more genes selected from the group consisting of: NXNL1, PSMA7, C6orf61, EMP1, CLIC1, LACTB and DUSP3 may be used as a means to determine the *M. tuberculosis* infection status in a subject.

One disclosure provides a gene expression product from a gene selected from the group consisting of NXNL1, PSMA7, C6orf61, EMP1, CLIC1, LACTB and DUSP3 or a gene selected from table 3 or table 4 for use as biomarker for infection by *M. tuberculosis.*

One disclosure provides an oligonucleotide capable of detecting the presence or expression level of a gene expression product from a gene selected from the group consisting of NXNL1, PSMA7, C6orf61, EMP1, CLIC1, LACTB and DUSP3 or a gene selected from table 3 or table 4 in a sample from a subject.

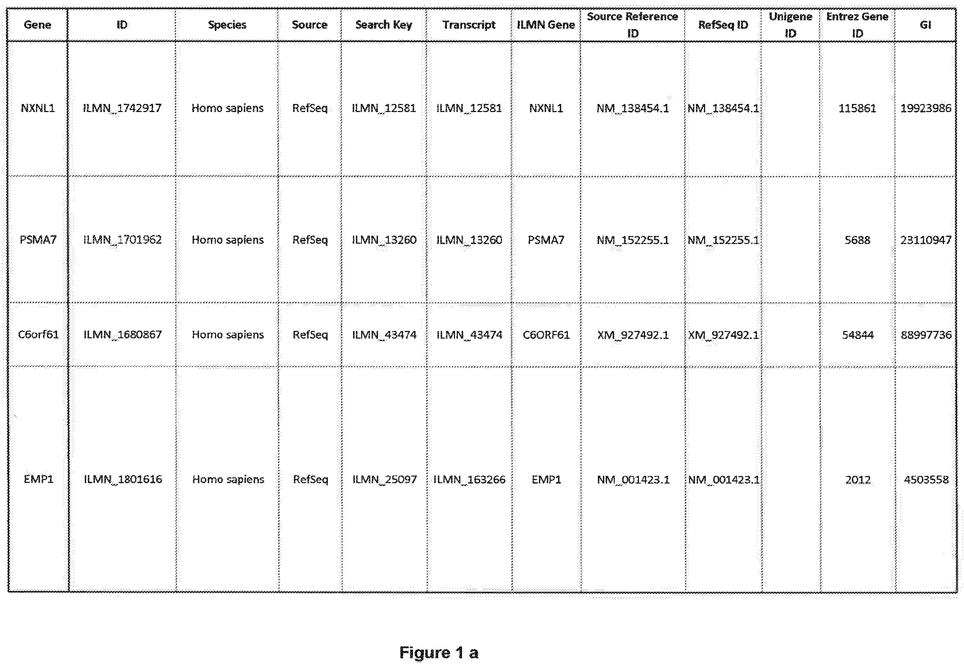

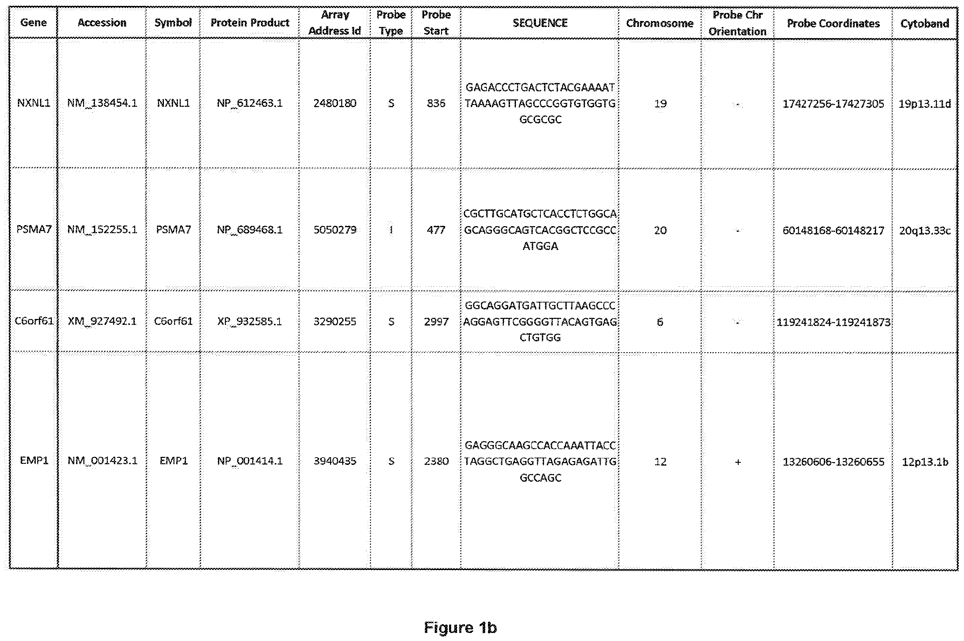

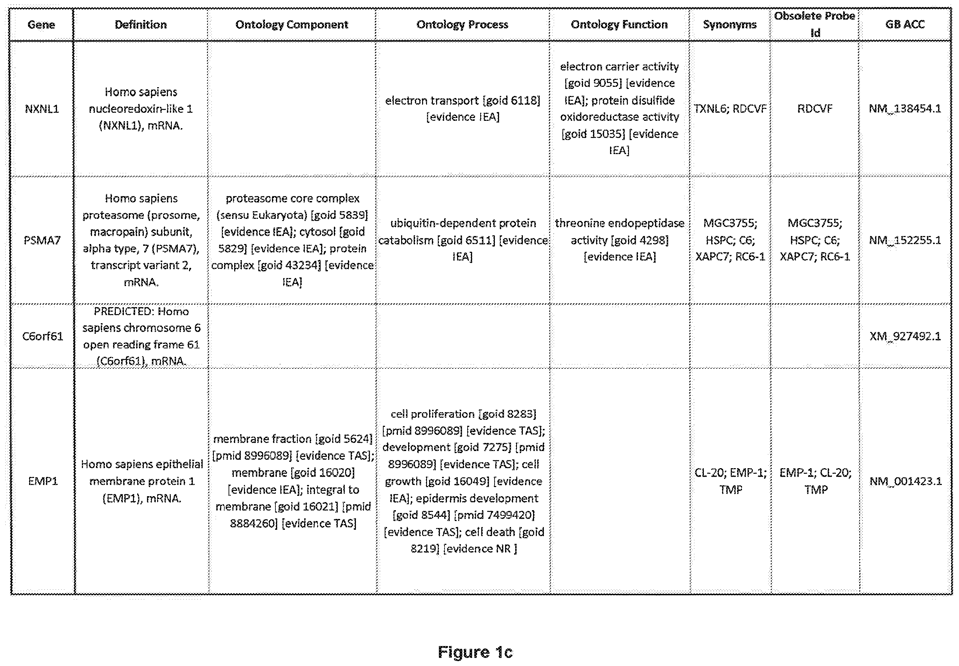

The nucleotide sequences shown for each gene in Figure 1 and Figure 2, Figure 3 and Figure 4 are sections of the nucleotide sequence for each gene that are particularly useful as representative sequences for diagnosis. Therefore it is particularly advantageous to design a pair of primers for identification of each gene that would bind within the sequence given for that gene in Figure 1, Figure 2, Figure 3 or Figure 4. The primers may be designed to amplify part of all of the given nucleotide sequence.

One disclosure provides a method, kit, use, gene or oligonucleotide as described herein with reference to the examples.

The use of an altered expression level of one or more genes selected from: CLIC1, LACTB and DUSP3 to differentiate between subjects with active *M. tuberculosis* infection and subjects with latent *M. tuberculosis* infection or the use of an altered expression level of one or more genes selected from: NXNL1, PSMA7, C6orf61 and EMP1 to differentiate between subjects not infected with *M. tuberculosis* and subjects with latent *M. tuberculosis* infection has many applications positively impacting on clinical care. It permits both the regular screening of susceptible populations and also the testing of all individuals who are suspected of *M. tuberculosis* infection, and thus enables more timely eradication treatment for initial infection, possibly preventing chronic infection. It also permits a quantitative assessment of the efficacy of antibiotic therapy resulting in interventions that are customised to the response of the individual patient. It also provides a tool for widespread use in epidemiological studies, an important consideration *M. tuberculosis* infection becomes more prevalent.

NXNL1, PSMA7, C6orf61, EMP1, CLIC1, LACTB and DUSP3 and a gene selected from table 3 or table 4 may be targets for development of new therapeutics for treating *M. tuberculosis* infection.

The skilled man will appreciate that preferred features of any one embodiment and/or aspect of the invention may be applied to all other embodiments or aspects of the invention.

To the extent that features of the figures and examples fall outside of the scope of the claims, they are included here only for illustration purposes.

The present invention will be further described in more detail, by way of example only, with reference to the following figures in which:
**Figure 1** - shows a table of information about the panel of biomarkers that are useful in differentiating between subjects that are not infected with *M*. *tuberculosis* and subjects with latent *M. tuberculosis* infection, these genes are: Homo sapiens nucleoredoxin-like 1 (NXNL1); Homo sapiens proteasome (prosome, macropain) subunit, alpha type, 7 (PSMA7); Homo sapiens chromosome 6 open reading frame 61 (C6orf61); and Homo sapiens epithelial membrane protein 1 (EMP1). The RNA sequences shown are mRNA sequences.

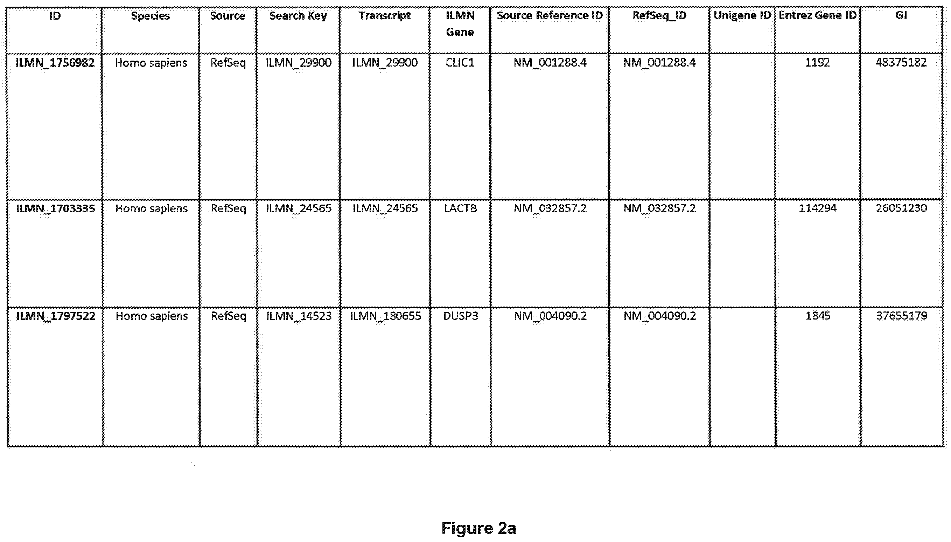

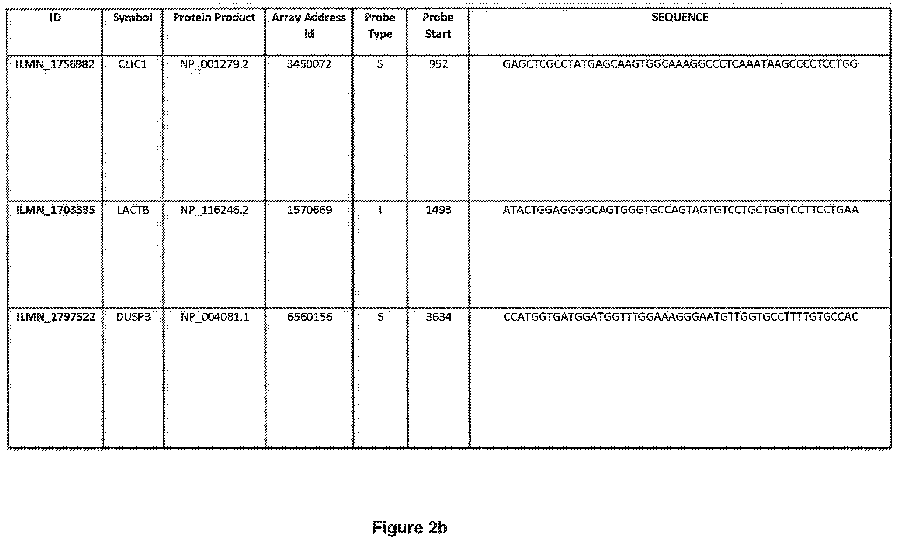

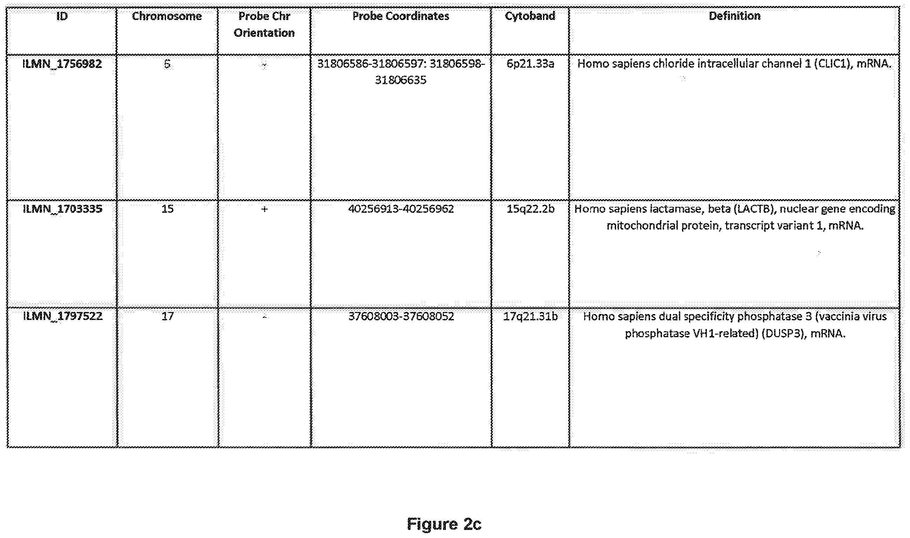

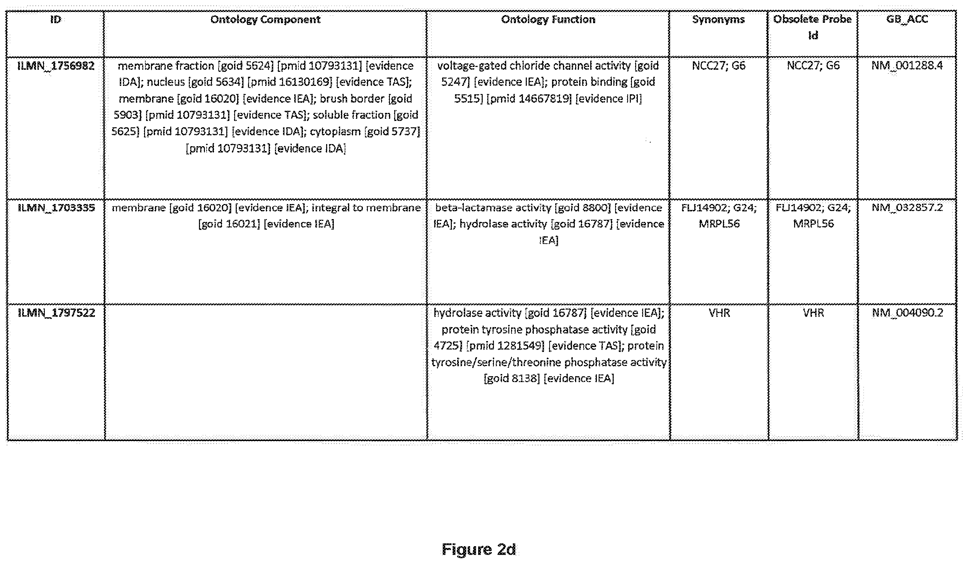

**Figure 2** shows a table of information about the panel of biomarkers that are useful in differentiating between subjects that have active *M*. *tuberculosis* infection and subjects with latent *M. tuberculosis* infection, these genes are: homo sapiens chloride intracellular channel 1 (CLIC1); Homo sapiens lactamase, beta (LACTB, nuclear gene encoding mitochondrial protein, transcript variant 1; and Homo sapiens dual specificity phosphatise 3 (vaccinia virus phosphatise VH1-related)(DUSP3). The RNA sequences shown are mRNA sequences.

**Figure 3** - shows a table of information about the panel of additional biomarkers that are useful in differentiating between subjects that are not infected with *M. tuberculosis* and subjects with latent *M. tuberculosis* infection, column 6 shows a sequence that is useful in identifying the gene and column 8 shows whether the gene is up or down regulated in subjects with latent *M. tuberculosis* infection.
**Figure 4** - shows a table of information about the panel of biomarkers that are useful in differentiating between subjects that have active *M*. *tuberculosis* infection and subjects with latent *M. tuberculosis* infection, column 6 shows a sequence that is useful in identifying the gene and column 8 shows whether the gene is up or down regulated in subjects with active *M*. *tuberculosis* infection.

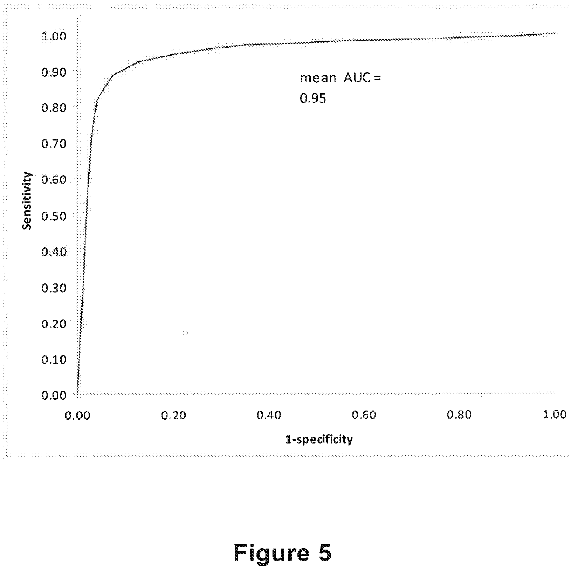

**Figure 5** - shows a ROC curve for the core set of biomarkers to distinguish between subjects that are not infected with *M. tuberculosis* and subjects with latent *M*. *tuberculosis* infection (NXNL1, PSMA7, C6orf61 and EMP1), ROC curves show the performance of the classifier incorporating both sensitivity and specificity. The higher the area under the ROC curve the better the performance of the classifier.

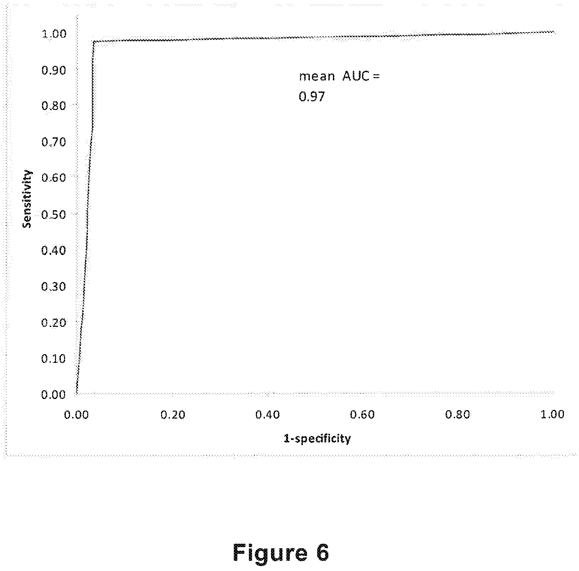

**Figure 6** - shows a ROC curve for the core set of biomarkers to distinguish between subjects that have active *M. tuberculosis* infection and subjects with latent *M*. *tuberculosis* infection (CLIC1, LACTB and DUSP3), ROC curves show the performance of the classifier incorporating both sensitivity and specificity. The higher the area under the ROC curve the better the performance of the classifier.

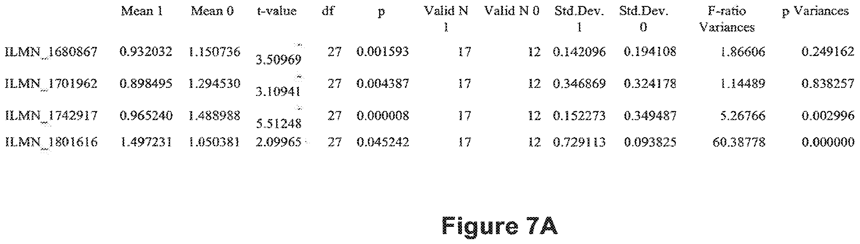

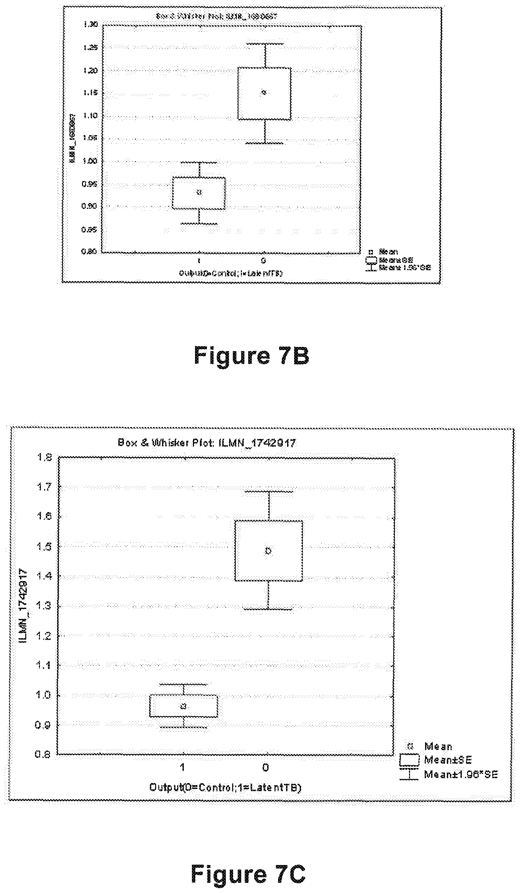

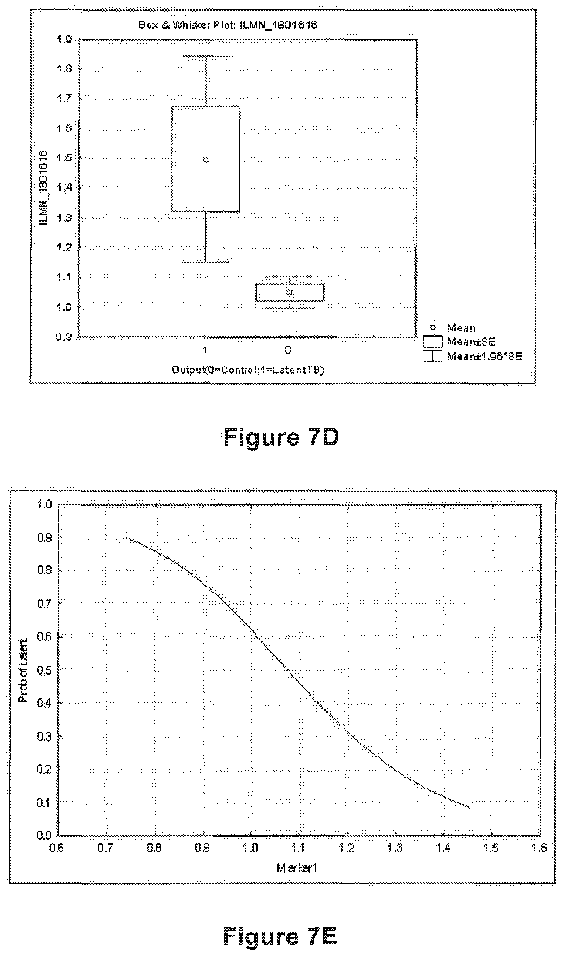

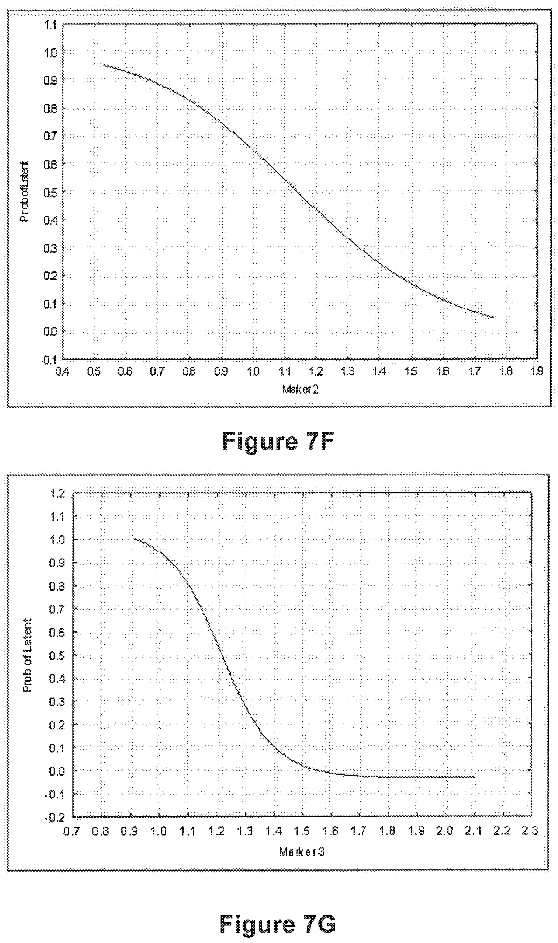

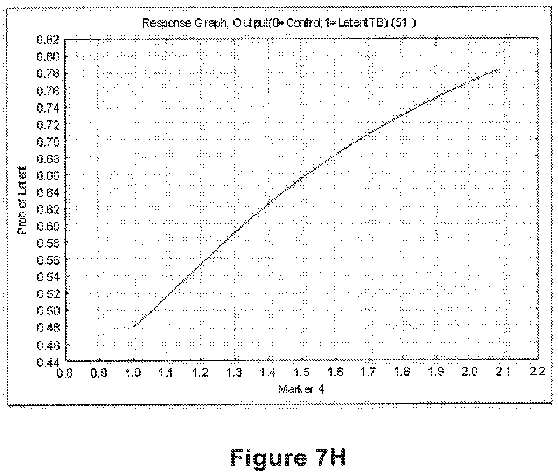

**Figure 7** - shows a stepwise summary for the core set of biomarkers to distinguish between subjects that are not infected with *M. tuberculosis* and subjects with latent *M. tuberculosis* infection (NXNL1, PSMA7, C6orf61 and EMP1). Markers are added in a stepwise fashion to build an optimised panel for classification.

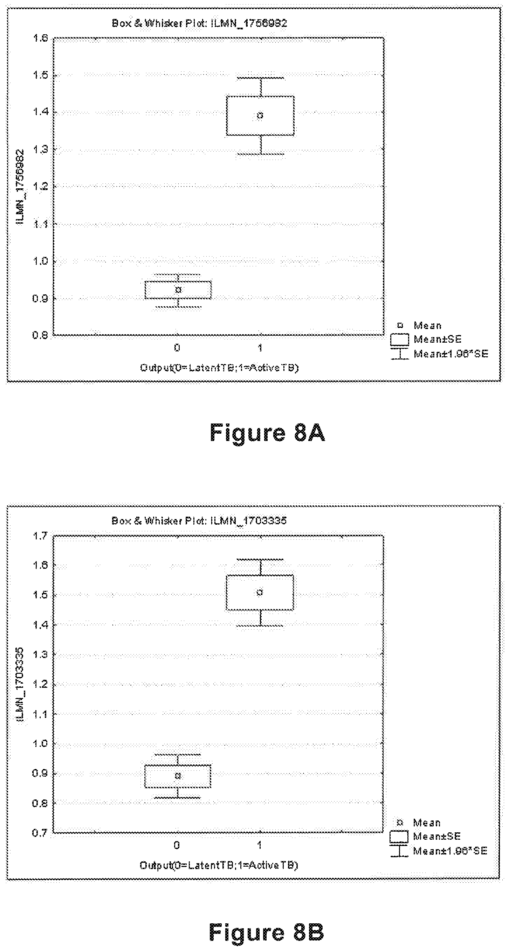

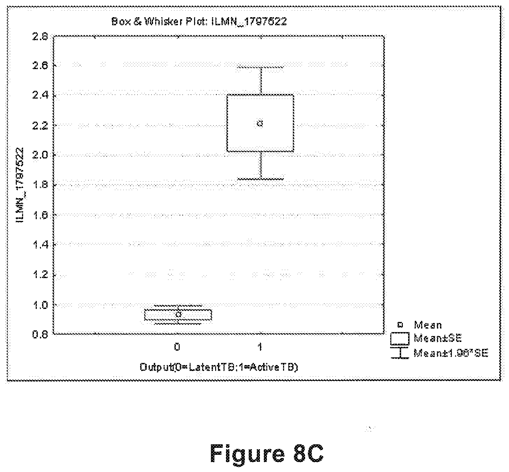

**Figure 8** - shows a stepwise summary for the core set of biomarkers to distinguish between subjects that have active *M. tuberculosis* infection and subjects with latent *M*. *tuberculosis* infection (CLIC1, LACTB and DUSP3) Markers are added in a stepwise fashion to build an optimised panel for classification.

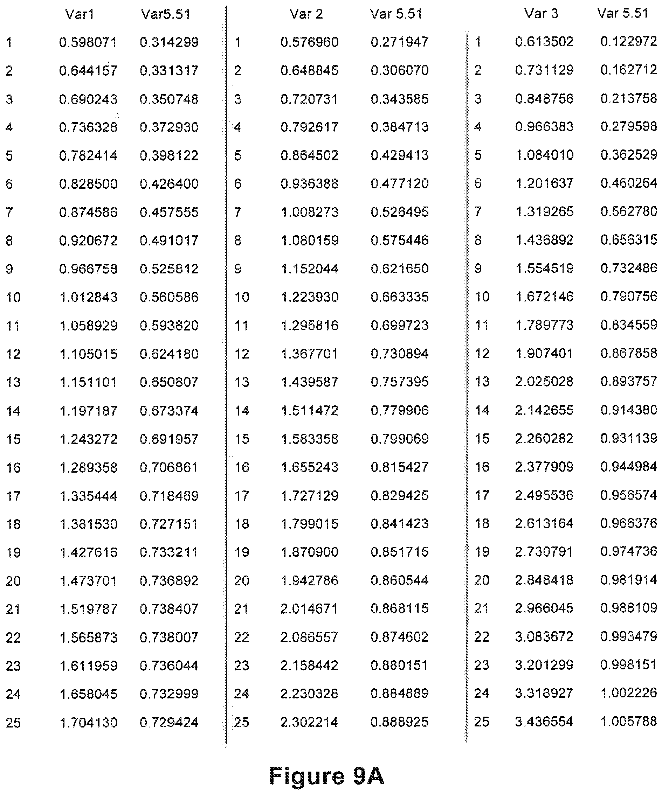

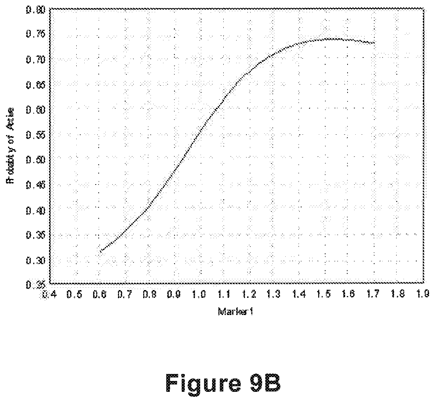

**Figure 9** - shows response data and curve for the core set of biomarkers to distinguish between subjects that have active *M. tuberculosis* infection and subjects with latent *M*. *tuberculosis* infection (CLIC1, LACTB and DUSP3) relating the expression level of the marker to the probability of class membership.

Raw data used to produce gene panes was taken from the GENE EXPRESSION OMNIBUS database Code: E-GEOD-22098.

The data was analysed using a data mining algorithm and method both described in patent application number PCT/GB2009/051412 published as: WO2010046697 and claiming priority to GB 0819221.3. This method provided two panels of biomarkers.
1) A panel of biomarkers that are useful in differentiating between subjects that are not infected with *M. tuberculosis* and subjects with latent *M. tuberculosis* infection, these genes are: Homo sapiens nucleoredoxin-like 1 (NXNL1); Homo sapiens proteasome (prosome, macropain) subunit, alpha type, 7 (PSMA7); Homo sapiens chromosome 6 open reading frame 61 (C6orf61); and Homo sapiens epithelial membrane protein 1 (EMP1).
2) A panel of biomarkers that are useful in differentiating between subjects that have active *M. tuberculosis* infection and subjects with latent *M. tuberculosis* infection, these genes are: homo sapiens chloride intracellular channel 1 (CLIC1); Homo sapiens lactamase, beta (LACTB, nuclear gene encoding mitochondrial protein, transcript variant 1; and Homo sapiens dual specificity phosphatise 3 (vaccinia virus phosphatise VH1-related) (DUSP3).

Tables 1 shows the log of mean expression value for each gene in subjects 10 with latent M. tuberculosis infection and Control subjects not infected with M. tuberculosis and the p-value showing the statistical significance of the difference.

**Table 1 - Control v Latent**

| | NXNL1 | PSMA7 | C6orf61 | EMP1 |
|---|---|---|---|---|
| Latent TB | 0.93 | 0.9 | 0.97 | 1.50 |
| Control uninfected | 1.15 | 1.29 | 1.49 | 1.05 |
| P-value | 0.0016 | 0.0044 | 0.8x10⁻⁵ | 0.045 |

Tables 2 shows the log of mean expression value for each gene in subjects with latent M. tuberculosis infection and subjects with active M. tuberculosis infection and the p-value showing the statistical significance of the difference.

**Table 2 -Latent v Active**

| | CLIC1 | LACTB | DUSP3 |
|---|---|---|---|
| Latent TB | 0.92 | 0.90 | 0.9 |
| Active TB | 1.40 | 1.5 | 2.2 |
| P-value | 0.4x10⁻⁵ | 0.14x10⁻⁵ | 0.1x10⁻⁶ |

**Table 3 - Control versus Latent probes of significance**

| ILMN_Gene | Definition |
|---|---|
| LOC389541 | Homo sapiens similar to CG14977-PA (LOC389541), mRNA |
| MID1IP1 | Homo sapiens MID1 interacting protein 1 (gastrulation specific G12 homolog (zebrafish)) (MID1IP1), mRNA |
| KLRC3 | Homo sapiens killer cell lectin-like receptor subfamily C, member 3 (KLRC3), transcript variant 1, mRNA |
| KLF9 | Homo sapiens Kruppel-like factor 9 (KLF9), mRNA |
| GPR177 | Homo sapiens G protein-coupled receptor 177 (GPR177), transcript variant 1, mRNA |
| FBXO32 | Homo sapiens F-box protein 32 (FBXO32), transcript variant 2, mRNA. |
| TAZ | Homo sapiens tafazzin (cardiomyopathy, dilated 3A (X-linked); endocardial fibroelastosis 2; Barth syndrome) (TAZ), transcript variant 3, mRNA |
| C5ORF29 | Homo sapiens chromosome 5 open reading frame 29 (C5orf29), mRNA |
| HSDL1 | Homo sapiens hydroxysteroid dehydrogenase like 1 (HSDL1), mRNA. |
| CHUK | Homo sapiens conserved helix-loop-helix ubiquitous kinase (CHUK), mRNA |
| LOC652062 | PREDICTED: Homo sapiens similar to Mitochondrial carnitine/acylcarnitine carrier protein (Carnitine/acylcarnitine translocase) (CAC) (LOC652062), mRNA |
| HIP1 | Homo sapiens huntingtin interacting protein 1 (HIP1), mRNA |
| C6ORF60 | Homo sapiens chromosome 6 open reading frame 60 (C6orf60), transcript variant 1, mRNA |
| MTMR11 | Homo sapiens myotubularin related protein 11 (MTMR11), transcript variant 1, mRNA. |

**Table 4 - Active versus Latent probes of significance**

| ILMN_Gene | Definition |
|---|---|
| LACTB | Homo sapiens lactamase, beta (LACTB), nuclear gene encoding mitochondrial protein, transcript variant 1, mRNA. |
| SRBD1 | Homo sapiens S1 RNA binding domain 1 (SRBD1), mRNA. |
| DUSP3 | Homo sapiens dual specificity phosphatase 3 (vaccinia virus phosphatase VH1-related) (DUSP3), mRNA. |
| ATG3 | Homo sapiens ATG3 autophagy related 3 homolog (S. cerevisiae) (ATG3), mRNA. |
| JAK2 | Homo sapiens Janus kinase 2 (a protein tyrosine kinase) (JAK2), mRNA. |
| PSMB8 | Homo sapiens proteasome (prosome, macropain) subunit, beta type, 8 (large multifunctional peptidase 7) (PSMB8), transcript variant 2, mRNA. |
| PSME1 | Homo sapiens proteasome (prosome, macropain) activator subunit 1 (PA28 alpha) (PSME1), transcript variant 1, mRNA. |
| ACOT9 | Homo sapiens acyl-CoA thioesterase 9 (ACOT9), transcript variant 2, mRNA. |
| IFI30 | Homo sapiens interferon, gamma-inducible protein 30 (IFI30), mRNA. |
| SORT1 | Homo sapiens sortilin 1 (SORT1), mRNA. |
| GSR | Homo sapiens glutathione reductase (GSR), mRNA. |
| TAP1 | Homo sapiens transporter 1, ATP-binding cassette, sub-family B (MDR/TAP) (TAP1), mRNA. |
| GRN | Homo sapiens granulin (GRN), mRNA. |
| IRF1 | Homo sapiens interferon regulatory factor 1 (IRF1), mRNA. |

### Sequences used in the application

**Table 5 -gene names and SEQ ID NO**

| ILMN Gene (names shown in figures 3 and 4) | SEQ ID NO | Sequence used to identify the gene |
|---|---|---|
| NXNL1 | 1 | GAGACCCTGACTCTACGAAAATTAAAAGTT AGCCCGGTGTGGTGGCGCGC |
| PSMA7 | 2 | CGCTTGCATGCTCACCTCTGGCAGCAGGGC AGTCACGGCTCCGCCATGGA |
| C6ORF61 | 3 | GGCAGGATGATTGCTTAAGCCCAGGAGTTC GGGGTTACAGTGAGCTGTGG |
| EMP1 | 4 | GAGGGCAAGCCACCAAATTACCTAGGCTGA GGTTAGAGAGATTGGCCAGC |
| CLIC1 | 5 | GAGCTCGCCTATGAGCAAGTGGCAAAGGCC CTCAAATAAGCCCCTCCTGG |
| LACTB | 6 | ATACTGGAGGGGCAGTGGGTGCCAGTAGTG TCCTGCTGGTCCTTCCTGAA |
| DUSP3 | 7 | CCATGGTGATGGATGGTTTGGAAAGGGAAT GTTGGTGCCTTTTGTGCCAC |
| LOC389541 | 8 | TGTGGTGAAGAGGCAGAACCGAGGTCGGG AGCCCATTGATGTCTGAGCCT |
| MID1IP1 | 9 | CCCCAGTGTGTATAAGCTGGCATTTCGCCA GCTTGTACGTAGCTTGCCAC |
| KLRC3 | 10 | GGGAAGAGAGTTTGCAGGCCTGTGCTTCAA AGAACTCTTCTAGTCTGCTT |
| KLF9 | 11 | GCCCTTCACCATTGTGGAATGATGCCCTGG |
| | | CTTTAAGGTTTAGCTCCACA |
| GPR177 | 12 | TGAGATCTACAAGTTGACCCGCAAGGAGGC CCAGGAGTAGGAGGCTGCAG |
| FBXO32 | 13 | GAAGGGTCCCCCTGCTGACTGGAGAGCTGG GAATATGGCATTTGGACACT |
| TAZ | 14 | GACAGATTTGTTCATAGACCCTCTCAAGTG CCCTCTCCGAGCTGGTAGGC |
| C5ORF29 | 15 | GCCTCTTCTCTCAAGCCTGCTTCAGATCATA AGTTCTTCCACACATCTCC |
| HSDL1 | 16 | GTGCCGAGCTGTCCATAGCTGCAGTGAAAG GTGAAGAGCAAGACCTTCTC |
| C6ORF60 | 17 | GCTTCTCCAGATCCCCAGCGCCAGGAGTGG TTTGCCCGGTACTTCACATT |
| CHUK | 18 | CCTTTATTTTGCTGCTTGATGATGAGAGGG AGGGCTGCTGCCACAGACTG |
| LOC652062 | 19 | ATGGAGCTGTCTTTCAGATCTTTCCTGGGAT TACCCCTGCCTACCCCCAG |
| HIP1 | 20 | TGCAGCCGTCCATAGCAGTACCCCTAAAAT CCCACCAGAATACGGGTCCC |
| MTMR11 | 21 | CACCAGAAGTAGCAGAGAAGCAGGGGCC AGAGCTACAACAGTATTCTTC |
| SRBD1 | 22 | ACTTCTACTTGCCAACATCTGCCTTGCTGGA CTTGTATGGGATTGTCTCC |
| ATG3 | 23 | AGTGACCATTGAAAATCACCCTCATCTGCC ACCACCTCCCATGTGTTCAG |
| JAK2 | 24 | TTGTCATCCTTTGAGCTGCTGACTGCCAATA ACATTCTTCGATCTCTGGG |
| PSMB8 | 25 | GTCGCTCGGACCCAGGACACTACAGTTTCT CTATGCGATCTCCAGAGCTC |
| PSME1 | 26 | ACCGGGACATCCGGCTGATGGTCATGGAGA TCCGCAATGCTTATGCTGTG |
| ACOT9 | 27 | GGACATTAAGTTCAGTGGCCATGTTAGCTG GGTCGGGAAGACATCCATGG |
| IFI30 | 28 | TGGAAGATCAGACCCAGCTCCTTACCCTTG |
| | | TCTGCCAGTTGTACCAGGGC |
| LACTB | 29 | ATACTGGAGGGGCAGTGGGTGCCAGTAGTG TCCTGCTGGTCCTTCCTGAA |
| DUSP3 | 30 | CCATGGTGATGGATGGTTTGGAAAGGGAAT GTTGGTGCCTTTTGTGCCAC |
| SORT1 | 31 | GGTCCCCATGTGCCTGTTGTTCAGCCCTCTC TCTTGTTCCCTTTCTGAGC |
| GSR | 32 | GAACCAGGAGACACGTGTGGCGGGCAGTG GGACCCATAGATCTTCTGAAA |
| TAP1 | 33 | GTAACGGAGTTTAGAGCCAGGGCTGATGCT TTGGTGTGGCCAGCACTCTG |
| GRN | 34 | AAGGCTCGATCCTGCGAGAAGGAAGTGGTC TCTGCCCAGCCTGCCACCTT |
| IRF1 | 35 | CCTCAACAGGCCCAGGGAGGGAAGTGTGA GCGCCTTGGTATGACTTAAAA |

## Claims

1. A method of determining the *M. tuberculosis* infection status of a subject comprising determining the expression level of NXNL1 and one or more genes selected from the group consisting of: PSMA7, C6orf61 and EMP1 in a sample from a subject,
wherein a decreased expression level of the gene NXNL1; and a decreased expression level of PSMA7 or C6orf61 or an increased expression level of the gene EMP1, differentiates between subjects not infected with *M. tuberculosis* and subjects with latent *M. tuberculosis* and indicates that a subject has a latent *M. tuberculosis* infection,
wherein the sample is a sample of blood, optionally whole blood, blood plasma or blood serum; sputum; saliva; wound exudate; urine; faeces; peritoneal fluid; or a respiratory section.

2. The method according to claim 1 further comprising employing the expression level determined to distinguish between subjects not infected with *M. tuberculosis* and subjects with latent *M. tuberculosis* infection.

3. The method according to any one of the preceding claims wherein the level of at least NXNL1 and PSMA7 are determined.

4. The method according to any one of the preceding claims wherein the level of at least NXNL1, PSMA7 and C6orf61 are determined.

5. The method according to any one of the preceding claims wherein the level of least at NXNL1, PSMA7, C6orf61 and EMP1 are determined.

6. The method according to any one of the preceding claims further comprising determining the expression level of one or more of the following genes:
LOC389541, MID1IP1, KLRC3, KLF9, FBXO32, C5ORF29, CHUK, LOC652062, C6ORF60, MTMR11, GPR117, TAZ, HSDL1 or HIP1.

7. The method according to any one of the preceding claims for use in one or more of the following; diagnosing whether or not a subject has *M. tuberculosis* infection; advising on the prognosis for a subject with a *M. tuberculosis* infection; and monitoring the effectiveness or response of a subject to a particular treatment for infection by *M. tuberculosis.*

8. The method according to any one of the preceding claims wherein
a decreased expression level of the gene NXNL1; and
a decreased expression level of PSMA7 or C6orf61 or an increased expression level of the gene EMP1; and at least one of:
increased expression of LOC389541, MID1IP1, KLRC3, KLF9, FBXO32, C5ORF29, CHUK, LOC652062, C6ORF60 or MTMR11, decreased expression GPR117, TAZ, HSDL1 or HIP1,
indicates that a subject has latent *M. tuberculosis* infection.

9. The method according to any one of the preceding claims wherein
a decreased expression level of the gene NXNL1; and
a decreased expression level of PSMA7 or C6orf61 or an increased expression level of the gene EMP1; and one, two or three of:
increased expression of LOC389541, MID1IP1, KLRC3, KLF9, FBXO32, C5ORF29, CHUK, LOC652062, C6ORF60 or MTMR11, decreased expression GPR117, TAZ, HSDL1 or HIP1,
indicates that a subject has latent *M. tuberculosis* infection.

10. The method according to any one of the preceding claims wherein a decreased expression level of the genes NXNL1, PSMA7 and C6orf61 and an increased expression level of the gene EMP1 and one, two or three of:
increased expression of LOC389541, MID1IP1, KLRC3, KLF9, FBXO32, C5ORF29, CHUK, LOC652062, C6ORF60 or MTMR11,
decreased expression GPR117, TAZ, HSDL1 or HIP1,
indicates that a subject has latent *M. tuberculosis* infection.

11. The method according to any one of the preceding claims wherein the altered expression level is an expression level that is higher or lower than the expression level expected in a subject not infected with *M. tuberculosis* and/or wherein the expression level of the gene is determined using the level of mRNA encoded by the selected gene present in the sample.

12. A method according to any one of the preceding claims wherein the expression level of the gene is determined using quantitative PCR.

13. Use of the determination of the expression level of NXNL1 and one or more genes selected from the group consisting of: PSMA7, C6orf61 and EMP1 as a means to determine the *M. tuberculosis* infection status in a subject.

## Patentansprüche

1. Verfahren zum Bestimmen des M. tuberculosis-Infektionsstatus eines Subjekts, umfassend das Bestimmen des Expressionsgrades von NXNL1 und eines oder mehrerer Gene, ausgewählt aus der Gruppe bestehend aus: PSMA7, C6orf61 und EMP1, in einer Probe von einem Subjekt,
wobei ein verminderter Expressionsgrad des Gens NXNL1; und ein verminderter Expressionsgrad von PSMA 7 oder C6orf61 oder ein erhöhter Expressionsgrad des Gens EMP1 zwischen nicht mit M. tuberculosis infizierten Subjekten und Subjekten mit latenter M. tuberculosis unterscheidet und anzeigt, dass ein Subjekt eine latente M. tuberculosis-Infektion hat,
wobei die Probe eine Blutprobe, gegebenenfalls Vollblut, Blutplasma oder Blutserum; Sputum; Speichel; Wundexsudat; Urin; Kot; Peritonealflüssigkeit; oder ein Atemwegsabschnitt ist.

2. Verfahren nach Anspruch 1, ferner umfassend das Verwenden des Expressionsgrades, der zum Unterscheiden zwischen nicht mit M. tuberculosis infizierten Subjekten und Subjekten mit latenter M. tuberculosis-Infektion bestimmt ist.

3. Verfahren nach einem der vorhergehenden Ansprüche, wobei der Grad an mindestens NXNL1 und PSMA7 bestimmt wird.

4. Verfahren nach einem der vorhergehenden Ansprüche, wobei der Grad an mindestens NXNL1, PSMA7 und C6orf61 bestimmt wird.

5. Verfahren nach einem der vorhergehenden Ansprüche, wobei der Grad an mindestens NXNL1, PSMA7, C6orf61 und EMP1 bestimmt wird.

6. Verfahren nach einem der vorhergehenden Ansprüche, ferner umfassend das Bestimmen des Expressionsgrades eines oder mehrerer der folgenden Gene:
LOC389541, MID1IP1, KLRC3, KLF9, FBXO32, C5ORF29, CHUK, LOC652062, C6ORF60, MTMR11, GPR117, TAZ, HSDL1 oder HIP1.

7. Verfahren nach einem der vorhergehenden Ansprüche zum Verwenden in einem oder mehreren der Folgenden; Diagnostizieren, ob ein Subjekt eine M. tuberculosis-Infektion hat oder nicht; Beraten über die Prognose für ein Subjekt mit einer M. tuberculosis-Infektion; und Überwachen der Wirksamkeit oder Reaktion eines Subjekts auf eine bestimmte Behandlung für eine Infektion durch M. tuberculosis.

8. Verfahren nach einem der vorhergehenden Ansprüche, wobei
ein verminderter Expressionsgrad des Gens NXNL 1; und
ein verminderter Expressionsgrad von PSMA7 oder C6orf61 oder ein erhöhter Expressionsgrad des Gens EMP1; und mindestens eines von:
erhöhter Expression von LOC389541, MID1IP1, KLRC3, KLF9, FBXO32, C5ORF29, CHUK, LOC652062, C6ORF60 oder MTMR11, verminderter Expression von GPR117, TAZ, HSDL1 oder HIP1
darauf hindeutet, dass ein Subjekt eine latente M. tuberculosis-Infektion hat.

9. Verfahren nach einem der vorhergehenden Ansprüche, wobei
ein verminderter Expressionsgrad des Gens NXNL1; und
ein verminderter Expressionsgrad von PSMA7 oder C6orf61 oder ein erhöhter Expressionsgrad des Gens EMP1; und eines, zwei oder drei von:
erhöhter Expression von LOC389541, MID1IP1, KLRC3, KLF9, FBXO32, C5ORF29, CHUK, LOC652062, C6ORF60 oder MTMR11, verminderter Expression GPR117, TAZ, HSDL1 oder HIP1
darauf hindeutet, dass ein Subjekt eine latente M. tuberculosis-Infektion hat.

10. Verfahren nach einem der vorhergehenden Ansprüche, wobei ein verminderter Expressionsgrad der Gene NXNL1, PSMA7 und C6orf61 und ein erhöhter Expressionsgrad des Gens EMP1 und eines, zwei oder drei von:
erhöhter Expression von LOC389541, MID1IP1, KLRC3, KLF9, FBXO32, C5ORF29, CHUK, LOC652062, C6ORF60 oder MTMR11,
verminderter Expression von GPR117, TAZ, HSDL1 oder HIP1
darauf hindeutet, dass ein Subjekt eine latente M. tuberculosis-Infektion hat.

11. Verfahren nach einem der vorhergehenden Ansprüche, wobei der veränderte Expressionsgrad ein Expressionsgrad ist, der höher oder niedriger ist als der bei einem nicht mit M. tuberculosis infizierten Subjekt erwartete Expressionsgrad und/oder wobei der Expressionsgrad des Gens unter Verwendung des Grades an mRNA bestimmt wird, für die durch das in der Probe vorhandene ausgewählte Gen kodiert wird.

12. Verfahren nach einem der vorhergehenden Ansprüche, wobei der Expressionsgrad des Gens mittels quantitativer PCR bestimmt wird.

13. Verwendung der Bestimmung des Expressionsgrades von NXNL1 und eines oder mehrerer Gene, ausgewählt aus der Gruppe bestehend aus: PSMA7, C6orf61 und EMP1, als Mittel zum Bestimmen des M. tuberculosis-Infektionsstatus bei einem Subjekt.

## Revendications

1. Procédé de détermination du statut de l'infection par *M*. *tuberculosis* chez un sujet, comprenant :
la détermination du niveau d'expression de NXNL1 et d'au moins un gène sélectionné dans l'ensemble constitué de : PSMA7, C6orf61 et EMP1 dans un prélèvement d'un sujet,
un niveau réduit d'expression du gène NXNL1 ; et un niveau réduit d'expression de PSMA7 ou de C6orf61 ou un niveau accru d'expression du gène EMP1 différentiant entre des sujets non infectés par *M*. *tuberculosis* et des sujets à *M*. *tuberculosis* latente, et indiquant qu'un sujet présente une infection latente par *M*. *tuberculosis,*
le prélèvement étant un prélèvement de sang, éventuellement de sang entier, de plasma sanguin ou de sérum sanguin ; de crachat ; de salive ; d'exsudat de lésion ; d'urine ; de matière fécale ; de fluide péritonéal ; ou d'une section respiratoire.

2. Procédé selon la revendication 1, comprenant en outre l'emploi du niveau d'expression pour distinguer entre les sujets non infectés par *M*. *tuberculosis* et les sujets à infection latente par *M*. *tuberculosis.*

3. Procédé selon l'une quelconque des revendications précédentes, dans lequel le niveau d'au moins NXNL1 et PSMA7 sont déterminés.

4. Procédé selon l'une quelconque des revendications précédentes, dans lequel le niveau d'au moins NXNL1, PSMA7 et C6orf61 sont déterminés.

5. Procédé selon l'une quelconque des revendications précédentes, dans lequel le niveau d'au moins NXNL1, PSMA7, C6orf61 et EMP1 sont déterminés.

6. Procédé selon l'une quelconque des revendications précédentes, comprenant en outre la détermination du niveau d'expression d'au moins un des gènes suivants :
LOC389541, MID1IP1, KLRC3, KLF9, FBXO32, C5ORF29, CHUK, LOC652062, C6ORF60, MTMR11, GPR117, TAZ, HSDL1 ou HIP1.

7. Procédé selon l'une quelconque des revendications précédentes, pour un usage dans au moins l'une des choses suivantes ; diagnostiquer si un sujet est atteint d'une infection par *M. tuberculosis* ; conseiller sur le pronostic pour un sujet atteint d'une infection par *M*. *tuberculosis* ; et suivre l'efficacité ou la réponse d'un sujet à un traitement particulier pour une infection par *M*. *tuberculosis.*

8. Procédé selon l'une quelconque des revendications précédentes, dans lequel un niveau réduit d'expression du gène NXNL1 ; et
un niveau réduit d'expression de PSMA7 ou de C6orf61 ou un niveau accru d'expression du gène EMP1 ; et au moins l'une des choses suivantes :
expression accrue de LOC389541, MID1IP1, KLRC3, KLF9, FBXO32, C5ORF29, CHUK, LOC652062, C6ORF60 ou MTMR11, expression réduite de GPR117, TAZ, HSDL1 ou HIP1, indique qu'un sujet est atteint d'une infection latente par *M*. *tuberculosis.*

9. Procédé selon l'une quelconque des revendications précédentes, dans lequel un niveau réduit d'expression du gène NXNL1 ; et
un niveau réduit d'expression de PSMA7 ou de C6orf61 ou un niveau accru d'expression du gène EMP1 ; et une, deux ou trois des choses suivantes :
expression accrue de LOC389541, MID1IP1, KLRC3, KLF9, FBXO32, C5ORF29, CHUK, LOC652062, C6ORF60 ou MTMR11, expression réduite de GPR117, TAZ, HSDL1 ou HIP1, indique qu'un sujet est atteint d'une infection latente par *M*. *tuberculosis.*

10. Procédé selon l'une quelconque des revendications précédentes, dans lequel un niveau réduit d'expression des gènes NXNL1, PSMA7 et C6orf61 et un niveau accru d'expression du gène EMP1 et une, deux ou trois des choses suivantes :
expression accrue de LOC389541, MID1IP1, KLRC3, KLF9, FBXO32, C5ORF29, CHUK, LOC652062, C6ORF60 ou MTMR11,
expression réduite de GPR117, TAZ, HSDL1 ou HIP1,
indique qu'un sujet est atteint d'une infection latente par *M*. *tuberculosis.*

11. Procédé selon l'une quelconque des revendications précédentes, dans lequel le niveau altéré d'expression est un niveau d'expression qui est supérieur ou inférieur au niveau d'expression attendu chez un sujet non infecté par *M*. *tuberculosis* et/ou dans lequel le niveau d'expression du gène est déterminé grâce au niveau d'ARNm codé par le gène sélectionné présent dans le prélèvement.

12. Procédé selon l'une quelconque des revendications précédentes, dans lequel le niveau d'expression du gène se détermine par PCR quantitative.

13. Usage de la détermination du niveau d'expression de NXNL1 et d'au moins un gène sélectionné dans l'ensemble constitué de : PSMA7, C6orf61 et EMP1 comme moyen de déterminer le statut de l'infection par *M*. *tuberculosis* chez un sujet.
